Europäisches Patentamt

European Patent Office

Office européen des brevets

(19)

(11) Publication number: 0 313 009
A2

## (12) EUROPEAN PATENT APPLICATION

(21) Application number: 88117380.1

(51) Int. Cl.⁴: C12N 15/00 , C12P 21/02

(22) Date of filing: 19.10.88

Claims for the following Contracting State: ES.

(30) Priority: 23.10.87 JP 266529/87

(43) Date of publication of application:
26.04.89 Bulletin 89/17

(84) Designated Contracting States:
AT BE CH DE ES FR GB GR IT LI LU NL SE

(71) Applicant: HOECHST JAPAN LIMITED
10-16, 8-chome, Akasaka, Minato-ku
Tokyo(JP)

(72) Inventor: Mizushima, Yuku
2-13-5-203 Shakujii-machi
Nerima-ku Tokyo(JP)
Inventor: Nakauchi, Hiromitsu
4-1-11 Ninomiya
Tsukuba-shi Ibaraki-ken(JP)
Inventor: Okumura, Ko
1-14-9, Matsunami
Chiba-shi Chiba-ken(JP)
Inventor: Watanabe, Hiroshi
1486-462, Ooza-Ishizaka Hatoyama-machi
Hiki-gun Saitama-ken(JP)
Inventor: Sugawara, Isamu
1-3-8 Kurihara
Niiza-shi Saitama-ken(JP)

(74) Representative: Meyer-Dulheuer,
Karl-Hermann, Dr. et al
HOECHST Aktiengesellschaft Zentrale
Patentabteilung Postfach 80 03 20
D-6230 Frankfurt/Main 80(DE)

(54) Method of gene amplification for enhancing protein production in cultured animal cells.

(57) Genes are effectively amplified in animal cells by transfecting the cells with the gene to be amplified and a hygromycin B-resistance gene and culturing the cells in a medium containing hygromycin B. The product encoded by the amplified gene is obtained in high yield.

EP 0 313 009 A2

# Method of gene amplification for enhancing protein production in cultured animal cells

Background of the Invention

1. Field of industrial use

The invention relates to a method of producing protein and animal cells used therefor.

According to the invention, various proteins, especially biologically active mammalian proteins which are useful as pharmaceuticals can be produced by culturing animal cells.

2. Description of prior art

Production of useful mammalian proteins by virtue of the gene-engineering techniques is mainly classified into two in terms of host cells, one is the use of a prokaryotic organism such as E. coli and the other is the use of mammalian cells such as Chinese hamster ovary cell lines (called CHO cells for short). Whereas the former is advantageous in that the desired protein is produced in a large amount, it is disadvantageous in that the biological activity and the stability are generally insufficient due to lack of the modifications of protein specific to animal cells. In the latter, whereas the desired protein is expected to be appropriately modified, the productivity per cell is rather low. In order to overcome the above-mentioned disadvantage of the latter, several systems have been developed in which genes coding for the desired proteins are introduced into animal cells, and the genes are amplified in the animal cells to increase the productivity per cell. As a well-known example of such a system there is a method in which a gene for dehydrofolate reductase (called dhfr for short), one of the enzymes essential for the biosynthesis of nucleic acids in cells, is introduced into dhfr-deficient cells together with the desired gene in a medium containing methotrexate (called MTX for short), a dhfr inhibitor, to amplify the dhfr gene and the desired gene simultaneously. Usually, the gene product of interest is produced in approximately 20% of the total dhfr(+) cells which can be acquired by an initial transfection. The copy number of the desired gene can be successively increased by increasing the concentration of MTX in the culture medium. The method, however, is beset with difficulties in that (1) it takes about one year to amplify the gene up to sufficiently high copy number to obtain a cell strain producing a sufficient amount of the gene product of interest, and (2) the dhfr-deficient animal cells available at present are practically limited to CHO cell lines. Use of other cell lines is often required depending upon the nature of the protein of interest.

Brief Description of the Drawing

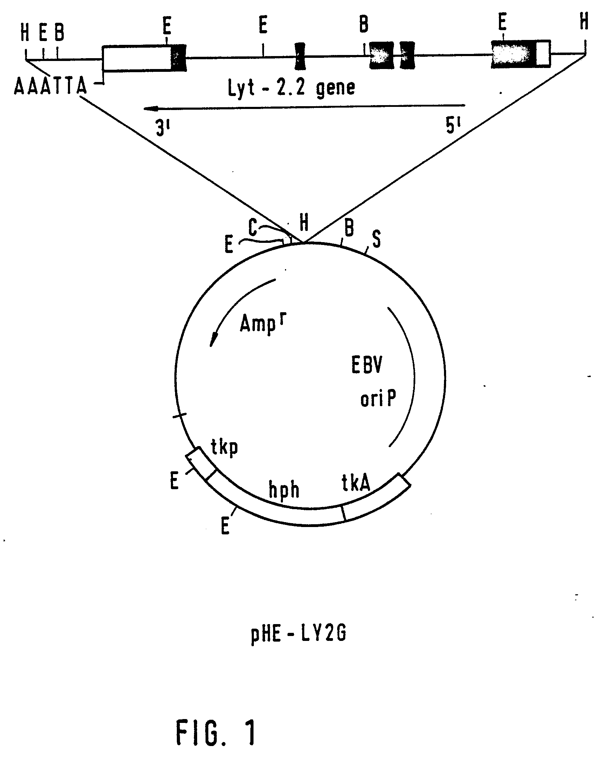

Fig.1 is a gene map of pHE-LY2G.

In the figure, H, E, B, C and S respectively indicate the restriction enzyme-recognition site for HindIII, EcoRI, BamHI, ClaI and SalI.

The white box and the black box in the Lyt-2.2 gene respectively represent a non-translated region and a translated region of the exon. Other regions of the Lyt-2.2 gene represent the intron and flanking regions on the 5′ and 3′ side of the gene. An AAATTA sequence adjacent to the exon located on the 3′ side represents the poly A additional signal. Amp$^r$ is an ampicillin resistant gene, and EBV oriP is the oriP region of Epstein-Barr virus. hph is a hygromycin B-resistant gene, and tkp and tkA respectively represent the promoter and the poly A signal of the thymidine kinase gene.

Summary of the Invention

The in vivo gene amplification according to the invention (1) overcomes the limitation that only a few enzyme-deficient cell lines such as dhfr(-) -CHO cell lines are available as host cells which are able to propagate the commonly used dhfr vector, and, consequently, the limit in a selective use of the most suitable cell line for the production of the protein of interest and (2) has the advantage that only 20-40 days are required for the gene introduced into the host cell to reach 20-50 copies per cell.

The invention relates to a method which comprises transfecting an animal cell with a gene coding for the protein of interest and a hygromycin B-resistant gene and growing the resulting transfectants in a hygromycin B-containing medium to increase the copy number of the gene coding for the protein of interest. Furthermore, the invention is concerned with a method of producing a protein by using animal cells with the genes thus increased.

Hygromycin B is an antibiotic produced by Streptomyces hygroscopicus which was discovered in 1953 by Pettinger et al. and has been placed in practical use as an anthelmintic for swine enteric parasites. The antibiotic inhibits protein synthesis both in eukaryotic cells and prokaryotic cells. The gene coding for hygromycin B phosphotransferase (called hph hereinbelow for short) which provides resistance to hygromycin B has been described by L. Gritz et al. for its base sequence and other characteristics (L. Gritz et al. Gene 25 (1983) 179-188). We have surprisingly found that the copy number of a protein-coding gene is increased by transfecting the protein-coding gene into animal cells together with the hph gene and growing the transfectants in a hygromycin B-containing culture medium and that this resulted in the increase of the protein productivity.

The above finding is very useful in that it is applicable not only to a limited scope of host cell lines such as dhfr(-) CHO cell lines but also, practically, to any kind of animal cells.

Many animal cell lines including insect cells may be used as the host cells, and preferred examples are:

| Ltk⁻ (ATCC CCL 1.3) | C3H mouse fibroblast L cell |
|---|---|
| BW5147.G.1.4.OVA$^R$ .1 (ATCC CRL 1588) | AKR/J mouse lymphoma origin |
| P 388 D$_1$ (ATCC TIB 63) | Mouse monocyte-macrophage origin |
| NIH/3T3 (ATCC CRL 1658) | NIH Swiss mouse fetal cell origin |
| THP-1 (ATCC TIB 202) | Human monocyte origin |
| U-937 (ATCC CRL 1593) | Human histiocyte lymphoma origin |
| Hep G2 (ATCC HB 8065) | Human hepatoma origin |
| FM3A | C3H/Hen mouse mammary cancer origin |
| Vero (ATCC CCL 81) | African green monkey kidney origin |
| CHO-K1 (ATCC CCL 61) | Chinese hamster ovary origin |
| BHK-21 (ATCC CCL 10) | Syrian hamster kidney origin |

The protein produced by the method of the invention covers all sorts of proteins produced by mammals. Examples highly useful from the medical point of view are as follows: Hormones such as insulin, growth hormones, parathyroid hormone, erythropoietin, nerve growth factors and LHRH (luteinizing hormone-releasing hormone); protease inhibitors such as tissue plasminogen activator, protein C and $\alpha_2$ plasmin inhibitor; blood coagulation factors such as Factor VIII and Factor IV; immunoglobulins; and other blood trace components.

In order to be expressed in animal cells the hph gene and the protein-coding gene used in the invention require a promoter for initiation of the translation and a region for the termination (poly A region, terminator, etc.). The gene coding for the protein of interest can originate from either a cDNA or a genomic DNA. These genes can be integrated into separate plasmids and can be co-transfected into a host cell. It is also feasible for both of the genes to be integrated into one plasmid and to be transfected into a host cell.

In principle all of the transfection methods are applicable including calcium phosphate co-precipitation, the polyethylene glycol method, microinjection, the pricking method, the DEAE-dextran method, the polybrene method and electroporation. The transfectant that has thus become hygromycin B resistant is grown with successively increasing concentrations of hygromycin B to increase the copy number of the gene coding for the protein of interest. The concentration of hygromycin B can be increased without particular limit provided that the transfectant can grow. Concentrations up to 5,000-10,000 μg/ml will not completely inhibit growth.

The method will be illustratively described below for the production of Lyt-2, a mouse suppressor T cell or killer T cell surface antigen which plays important roles in the immune system, by using mouse fibroblasts as the host. The Lyt-2 antigen which was found by H. Cantor et al. [J. Exp. Med., 141, 1376-1389 (1975); Immunol. Rev. 33, 105-124 (1977), etc.] consists of two allotypes, Lyt-2.1 which is found in the C3H mouse and Lyt-2.2 which is found in the Balb/c mouse or C57BL/6 mouse. In the forthcoming experiment, the 5.3 kbp HindIII fragment containing the entire genomic sequence of Lyt-2.2 was used.

As the host cell, the Ltk⁻ cell line (ATCC CCL 1.3) containing no thymidine kinase (tk) gene was used among the C3H mouse fibroblast L cells.

As the plasmid vector carrying the hygromycin B-resistant gene, pHEBo was used which was prepared by B. Sugden et al. [Mol. Cell. Biol., 5, 410-413 (1985)]. The pHEBo is a plasmid vector prepared for the expression in animal cells by integrating a DNA fragment containing the oriP of the Epstein-Barr virus of the type B 95-8, the base sequence of which was described by Baer [Nature 310, 207-211 (1984)], into the BstE II site of the plasmid vector pHyg, which was constructed from a DNA fragment contain-ing an ampicillin-resistant gene and ori from pBR322 and a DNA fragment containing hph originating from hygromycin B-resistant E. coli and inserted between a promoter and a poly A signal of the thymidine kinase (tk) gene of herpes simplex virus, type I.

Example Amplification of the Lyt-2 gene

Preparation of the gene for transfection

A HindIII 5.3 kbp fragment containing the entire genomic gene of Lyt-2.2 [H. Nakauchi et al., Proc. Natl. Acad. Sci. USA, 82, 5126-5130 (1985)] was subcloned at the HindIII site of pHEBo to construct pHE-LY2G. The pHE-LY2G was opened at the C1al site which was a single restriction site for linearizing the plasmid.

Introduction of the gene into host cells

Calcium phosphate co-precipitates were prepared by conventional methods in such a way that the final DNA concentrations became 50 ng/ml for the pHE-LY2G and 20 $\mu$g/ml for the calf thymus DNA used as carrier. Introduction of the gene was carried out by adding 1 ml of the co-precipitates to Ltk(-) cells which had been spread 20 hours before at a concentration of $1 \times 10^6$ cells per 10 cm plate in diameter with 8 ml of DME medium containing 10% fetal bovine serum. The DME medium was exchanged after one day, and on the following days the medium was exchanged every 3-4 days with DME medium containing 500 $\mu$g/ml of hygromycin B. After about 2 weeks, cells which had acquired resistance to hygromycin B formed colonies. Efficiency of the colony development was 200-2,000 per plate.

Confirmation of the expression of the Lyt-2 gene and establishment of the transfectant cell lines

Expression of Lyt-2 on the cell surface was analyzed using a fluorescence activated cell sorter (called FACS hereinbelow). The cells were reacted with rat anti-Lyt-2 antibody, mouse anti-Lyt-2.1 antibody or mouse anti-Lyt-2.2 antibody. All of them were labeled with fluorescein isothiocyanate (FITC) to confirm the existence of each antigen. In the case of untreated L cell of C3H mouse origin, the allotype of Lyt-2 is Lyt-2.1, but neither Lyt-2.1 nor Lyt-2.2 was detected. On the other hand, it was confirmed that the hygromycin B-resistant cells obtained by transfecting pHE-LY2G genes expressed Lyt-2.2 approximately in 50% of the total transfectants and expression of Lyt-2.1 was not observed in any of the cells. Establishment of cell lines was carried out by the limiting dilution method.

Amplification of the copy number of the gene by increasing the reagent concentration

All seven cell lines, clone number 1, 2, 3, 5, 6, 7 and 10 obtained by cloning the pHE-LY2G transfectants, were separated and grown in a 10% fetal calf serum-containing DME medium containing hygromycin B at a concentration of 0 $\mu$g/ml, 500 $\mu$g/ml, 1,500 $\mu$g/ml and 3,000 $\mu$g/ml. The period of time required for formation of a very small colony was about 3 days for the concentration of hygromycin B in the range of 0-1,500 $\mu$g/ml, and about 2 weeks for the concentration of 3,000 $\mu$g/ml. When the concentration of hygromycin B was 0-1,500 $\mu$g/ml and the number of cells reached $2 \times 10^7$ after 20 day of culture, the cultivation was stopped. When it was 3,000 $\mu$g/ml, the cultivation was stopped after about 40 days.

Confirmation of the amplification of the amount of Lyt-2.2 antigen expression

To assay the amount of Lyt-2.2 antigen produced on the cell surface, $1 \times 10^6$ cells of transfectants and

4

FITC- labeled antibody were used for FACS analysis.

Results of the FACS analysis indicate that the expressed amount of Lyt-2.2 antigen in all of the above seven cell lines increased depending upon the concentration of hygromycin B. Degrees of the increase are shown in Table 1 in terms of the mean value or the mode value.

In Table 1, relative fluorescence intensity is shown by the unit of fluorescence intensity of FITC which has the excitation wavelength at 488 nm and the maximum wavelength of emission spectrum at 535 nm. The mode value in the distribution of fluorescence intensity is the same as the peak value and the mean value represents the mathematical mean value of the distribution. The standardized mode value and the standardized mean value respectively represent the value of the corresponding fluorescence intensity when the mode value and the mean value at 500 μg/ml of hygromycin B for each clone are taken as 1, respectively.

Table 1

| Expressed amount of Lyt-2 antigen estimated from the fluorescene intensity of the transfectants with pHE-LY2G | | | | | |
|---|---|---|---|---|---|
| Name of the cell | Concentration of hygromycin B | Mode value | | Mean value | |
| | | Relative fluorescence intensity | Standardized mode value | Relative fluorescence intensity | Standardized mean value |
| Clone 1 | 0 | 3.0 | 0.01 | 18.6 | 0.34 |
| | 500 | 32.1 | 1 | 48.7 | 1 |
| | 1500 | 82.0 | 2.7 | 69.5 | 1.5 |
| Clone 2 | 0 | 2.3 | - | 16.0 | 0.3 |
| | 500 | 26.8 | 1 | 47.2 | 1 |
| | 1500 | 71.0 | 2.9 | 67.2 | 1.5 |
| | 3000 | 88.1 | 3.6 | 87.7 | 1.9 |
| Clone 3 | 0 | 1.0 | - | 10.7 | 0.19 |
| | 500 | 25.8 | 1 | 43.4 | 1 |
| | 1500 | 82.0 | 3.5 | 59.7 | 1.4 |
| | 3000 | 109.5 | 4.7 | 83.7 | 2.0 |
| Clone 5 | 0 | 0.1 | 0.17 | 18.4 | 0.33 |
| | 500 | 24.0 | 1 | 49.0 | 1 |
| | 1500 | 82.0 | 3.8 | 70.4 | 1.5 |
| | 3000 | 122.0 | 5.7 | 103.4 | 2.2 |
| Clone 6 | 0 | 3.5 | 0.04 | 15.3 | 0.30 |
| | 500 | 27.7 | 1 | 44.4 | 1 |
| | 1500 | 46.0 | 1.7 | 60.0 | 1.4 |
| | 3000 | 105.6 | 4.2 | 96.9 | 2.3 |
| Clone 7 | 0 | 3.3 | 0.04 | 14.0 | 0.27 |
| | 500 | 20.8 | 1 | 44.3 | 1 |
| | 1500 | 44.4 | 2.3 | 54.6 | 1.2 |
| | 3000 | 85.0 | 4.6 | 90.0 | 2.0 |
| Clone 10 | 0 | 5.7 | 0.16 | 10.9 | 0.25 |
| | 500 | 22.3 | 1 | 35.0 | 1 |
| | 1500 | 28.8 | 1.3 | 52.0 | 1.5 |
| | 3000 | 98.2 | 4.9 | 93.4 | 2.8 |
| L tk⁻ | 0 | 2.6 | 0 | 3.0 | 0 |

Confirmation of the amplification of Lyt-2 gene

To confirm the increase of the copy numbers of the gene, DNA was prepared by the following procedures from $2 \times 10^7$ cultured cells of each cell line. Extrachromosomal DNA of the cultured cell was prepared by the Hirt extraction method [B. Hirt, J. Mol. Biol. 26, 365-369 (1967)] followed by conventional chromosomal DNA preparation methods from the DNA fraction obtained as a precipitate.

The isolated DNA was first digested with HindIII which excises the Lyt-2 gene in a size of 5.3 kbp for Southern hybridization analysis. About 10 μg of DNA per lane was size-fractionated by electrophoresis using 0.75% agarose as a carrier.

After fixing the DNA by transfer to a nylon membrane, Lyt-2 gene was detected with a $^{32}$P-Lyt-2.2 cDNA probe labeled by the nick translation method. In order to correct the amount of DNA on each lane, Lyt-3 gene was employed as internal standard. In fact, the intensity of the Lyt-2 band was standardized by taking as 1 the band intensity on the autoradiography of Lyt-3 one copy of which was present in every cell.

Intensity of the band on the autoradiogram of the Southern hybridization method was measured quantitatively by a densitometer. Results are shown in Table 2. The relative value for each clone results when taking as 1 the number of the Lyt-2 gene obtained in the presence of 500 μg/ml of hygromycin B. Taking as 1 the copies of Lyt-2 in the L cells without the transfected gene, the number of the Lyt-2 genes in the transfectants was about 4-10 copies in the presence of 500 μg/ml of hygromycin B and 20-50 copies at 3,000 μg/ml.

When Southern hybridization was done for the same DNA sample as above using pHEBo as probe, copy numbers of pHEBo were also increased in parallel with the copy numbers of Lyt-2.

Table 2.

| Relative amount of Lyt-2.2 gene in the transfectant cells with pHE-LY2G | | | |
|---|---|---|---|
| Name of cell line | 0 | 500 | 1500 | 3000 (μg/ml) |
| clone 2 | 0.29 | 1 | 1.9 | 3.5 |
| clone 5 | - | 1 | 2.0 | 5.4 |
| clone 7 | 0.17 | 1 | 1.2 | 4.5 |

## Claims

1. A method of amplifying a gene in animal cells, characterized by transfecting said cell with said gene and a hygromycin B-resistance gene and culturing the cells in a medium containing hygromycin B.

2. A method according to claim 1, characterized in that the cells are mammalian cells.

3. A method according to claim 1 or 2, characterized in that the gene to be amplified and the resistance gene are introduced simultaneously.

4. An animal cell containing an increased copy number of a gene, obtained by a process according to one or more of the preceding claims.

5. The use of a cell according to claim 4 for the production of the protein encoded by the said gene.

Claims for the following Contracting State: ES

1. A method of amplifying a gene in animal cells, characterized by transfecting said cell with said gene and a hygromycin B-resistance gene and culturing the cells in a medium containing hygromycin B.

2. A method according to claim 1, characterized in that the cells are mammalian cells.

3. A method according to claim 1 or 2, characterized in that the gene to be amplified and the resistance gene are introduced simultaneously.

4. A method of producing a protein, characterized by culturing cells obtained according to one or more of the preceding lcaims and isolating the protein encoded by the amplified gene.

pHE - LY2G

FIG. 1